# EUROPÄISCHE PATENTSCHRIFT

(11) **EP 2 084 289 B1**
(45) Veröffentlichungstag und Bekanntmachung des Hinweises auf die Patenterteilung: **04.03.2015**
(21) Anmeldenummer: 07821668.6
(22) Anmeldetag: 23.10.2007
(51) Int. Cl.: C12P 7/62

(54) **VERFAHREN ZUR HERSTELLUNG VON EPOXYGRUPPEN ENTHALTENDEN (METH)ACRYLSÄUREESTERN UNTER VERWENDUNG VON LIPASEN**
PROCESS FOR PRODUCING OF EPOXY-CONTAINING (METH)ACRYLIC ESTERS, USING LIPASES
PROCÉDÉ DE FABRICATION D'ESTERS D'ACIDE (MÉTH)ACRYLIQUE CONTENANT DES GROUPEMENTS ÉPOXY EN UTILISANT DES LIPASES

(30) Priorität: 26.10.2006 EP 06123028
(43) Veröffentlichungstag der Anmeldung: 05.08.2009
(73) Patentinhaber: BASF SE, 67056 Ludwigshafen (DE)
(72) Erfinder: HÄRING, Dietmar, 68535 Neu-edingen (DE); MEISENBURG, Uwe, 68161 Mannheim (DE); CHABANAS, Mathieu, 76768 Berg (DE); LIPOWSKY, Gunter, 69198 Schriesheim (DE)
(86) Internationale Anmeldenummer: PCT/EP2007/061304
(87) Internationale Veröffentlichungsnummer: WO 2008/049814

(56) Entgegenhaltungen:
- WO-A-2004/042069
- XIN, D. ET AL.: "Synthesis of Glycidyl Acrylate in Organic Solvents Catalyzed by Lipase" SEN I GAKKAISHI, Bd. 52, Nr. 10, Oktober 1996 (1996-10), Seiten 524-528, XP008086837 in der Anmeldung erwähnt

## Beschreibung

Die vorliegende Erfindung betrifft ein Verfahren zur Herstellung von Epoxygruppen enthaltenden (Meth)acrylsäureestern und deren Verwendung.

Unter (Meth)acrylsäure im Sinne der vorliegenden Erfindung werden Acrylsäure und/oder Methacrylsäure verstanden, unter (Meth)acrylsäureester Acrylsäureester und/oder Methacrylsäureester.

Die Herstellung von (Meth)acrylsäureestern erfolgt zumeist durch säure- oder basenkatalysierte Veresterung von (Meth)acrylsäure oder Umesterung von anderen (Meth)acrylsäureestern mit Alkoholen.

(Meth)acrylsäureester von Epoxygruppen enthaltenden Alkoholen sind prinzipiell bekannt. Derartige Ester finden beispielsweise Anwendung in Polymerdispersionen für Lacke und Coatings

US 2,680,109 beschreibt die Herstellung von Polymeren aus Monomeren, die wenigstens eine 1,2-Epoxygruppe enthalten. Gemäß Beispiel 1 wurde Glycidylmethacrylat aus Methacrylchlorid und Glycidol in Gegenwart von Pyridin in Benzol synthetisiert. Nach der Destillation wurde ein Reinprodukt mit unbekannter Ausbeute erhalten.

Aus der US 2,556,075 ist ebenfalls ein Verfahren zur Herstellung von Polymeren mit Glycidyl-einheiten bekannt. Glycidyl(meth)acrylat wurde demnach aus Kalium(meth)acrylat und Epichlor-hydrin 23 Stunden bei 118 °C synthetisiert. Das Reinprodukt wurde nach Destillation in unbekannter Ausbeute erhalten.

Gladkikh et al. offenbaren in J. Org. Chem. USSR, 1975, 11, 1602-1604 die Herstellung von (2-Glycidyloxy)ethylacrylat aus Hydroxyethylacrylat und Epichlorhydrin in Benzol mit Bortrifluorid bei 70 °C und einer Reaktionszeit von einer Stunde. Nach Aufreinigung (Extraktion, Destillation, und Abreagieren der chlorhaltigen Verunreinigungen) wurde ein Reinprodukt mit 40 % Ausbeute erhalten.

In J. Biol. Chem., 1977, 252, 6631-6639 beschreiben Füller et al. die Synthese von (Butandiolglycidylglycerylether)acrylat durch Addition von Acrylsäure an Butandioldiglycidylether. Das dabei entstehende bräunliche Material wies eine Reinheit von 80 % auf und wurde für die Polymerisation weiter verwendet.

In JP 2004334506 wird die Synthese von Epoxy-terminierten (Meth)acrylaten durch Umesterung von Methacrylat mit Epoxy-terminierten Alkoholen mit titanhaltigen Katalysatoren wie beispielsweise Ti(OBu)₄ offenbart. Nach Extraktion mit Toluol und anschließender Destillation wurde das Produkt mit einer Ausbeute von 83 % in einer Reinheit von 99,5 % erhalten.

Ein Verfahren und die Herstellung von Hydroxyalkyl(meth)acrylaten ausgehend von Epoxygruppen aufweisenden Alkoholen werden ebenfalls in EP 1 693 359 A1 beschrieben. Die Umsetzung der Alkohole erfolgt in Gegenwart von Lewis Säuren, die jeweils mindestens eine unmittelbar gebundene Di(cyclo)alkylaminogruppe tragen.

Diesen genannten Syntheseverfahren ist allen gemeinsam, dass der Epoxygruppen enthaltende (Meth)acrylsäureester auf herkömmlich chemischem Wege erhalten wird.

WO 2004/042069 beschreibt ein Verfahren zur Herstellung von mit aktinischer Strahlung und/oder Dual-Cure-härtbarer Poly(meth)acrylate, wobei hydroxyfunktionelle Seitenketten des Poly(meth)acrylats mit einem (Meth)acrylat oder (Meth)acrylsäure in Anwesenheit einer Lipase um- oder verestert werden.

Von Xin et al. wurde in Seni Gakkaishi 1996, 52 (1), 524-528 erstmals die Lipasekatalysierte Synthese von Glycidylacrylat aus Glycidol und Vinylacrylat beschrieben. In dieser Arbeit wurde der Einfluss von drei verschiedenen Lipasen, vier Lösungsmitteln sowie polymeren Additiven auf den Umsatz untersucht. Quantitative Umsetzungen wurden nicht beschrieben, da die Reaktionen alle nach vier Stunden bei einem maximalen Umsatz von 75 % abgebrochen wurden. Beim eingesetzten Vinylacrylat ist die alkoholische Abgangsgruppe nicht stabil, da Vinylalkohol zu Acetaldehyd isomerisiert und dadurch das Reaktionsgleichgewicht nicht mehr auf die Seite der Edukte verschoben werden kann. Derartige Acrylsäurederivate sind wegen ihrer hohen Herstellkosten für eine wirtschaftliche Synthese nicht von Interesse.

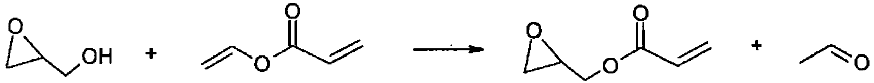

Aufgabe der vorliegenden Erfindung war es daher, ein Verfahren zur Verfügung zu stellen, mit dem (Meth)acrylsäureester von Epoxygruppen enthaltenden Alkoholen ausgehend von großtechnisch verfügbaren (Meth)acrylsäure und/oder (Meth)acrylsäureestern durch Ver- oder Umesterung erhalten werden. Das Verfahren soll ohne aufwendige Reinigungsschritte wie Extraktion oder Destillation des Produkts Reinheiten von mindestens > 99 % ergeben.

Die Aufgabe wurde gelöst durch ein Verfahren zur Herstellung von (Meth)acrylsäureestern (F) von mindestens eine Epoxygruppe aufweisenden Alkoholen (A), dadurch gekennzeichnet, dass man mindestens einen mindestens eine Epoxygruppe aufweisenden Alkohol (A) in Gegenwart mindestens eines Enzyms (E) mit (Meth)acrylsäure (S) verestert oder mit mindestens einem (Meth)acrylsäureester (D) umestert, wobei im Falle der Umesterung die alkoholische Abgangsgruppe unter den Reaktionsbedingungen stabil ist, wobei es sich bei dem (Meth)acrylsäureester (D) um einen gesättigten C₁ - C₁₀-Alkylester handelt, es sich bei dem Epoxygruppen aufweisenden Alkohol (A) um einen der Formel (1) 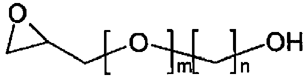 handelt,
worin m eine ganze Zahl und 0 oder 1 ist und n ebenfalls eine ganze Zahl von 0 bis 10, mit der Maßgabe, dass im Falle von m = 1 n ≠ 0 ist und das Enzym (E) ausgewählt ist aus der Gruppe der Lipasen (E.C. 3.1.1.3).

Im Folgenden werden die Edukte (Meth)acrylsäure (S) und (Meth)acrylsäureester (D) auch gemeinsam unter dem Begriff (Meth)acrylverbindung (B) zusammengefasst.

Mit Hilfe des erfindungsgemäßen Verfahrens ist die Herstellung derartiger (Meth)acrylsäureester (F) in hoher chemischer und Raum-Zeit-Ausbeute und unter milden Bedingungen mit guten Farbzahlen unter Verzicht auf Schutzgruppenoperationen und unter Einsatz einfacher Ausgangsstoffe möglich.

Die enzymatisch katalysierte Herstellung von Epoxygruppen enthaltenden (Meth)acrylsäureestern (F) erfolgt unter milderen Reaktionsbedingungen als mit aus dem Stand der Technik bekannten chemischen Veresterungskatalysatoren.

Erfindungsgemäß geeignete Alkohole (A) sind solche Alkohole, die mindestens eine Epoxygruppe und mindestens eine Hydroxygruppe enthalten.

Beispielsweise können solche Alkohole 1 bis 3, bevorzugt 1 bis 2 und besonders bevorzugt genau eine Epoxygruppe enthalten.

Die Alkohole (A) können ein bis sechs, bevorzugt ein bis vier, besonders bevorzugt ein bis drei, ganz besonders bevorzugt ein bis zwei und insbesondere genau eine Hydroxygruppe enthalten.

Besonders bevorzugt sind solche Alkohole (A), die genaue eine Epoxygruppe und genau eine Hydroxygruppe enthalten.

Die erfindungsgemäß einsetzbaren Alkohole (A) können noch andere Heteroatome wie beispielsweise Stickstoff, Sauerstoff und Schwefel enthalten, bevorzugt sind sie nur aus Kohlenstoff-, Wasserstoff- und Sauerstoffatomen aufgebaut.

Die erfindungsgemäß einsetzbaren Alkohole (A) können noch andere funktionelle Gruppen enthalten, beispielsweise C-C-Doppelbindungen, Amino-, Carboxy-, Ether-oder Carbonsäureestergruppen.

Die Hydroxygruppen der erfindungsgemäß einsetzbaren Alkohole (A) können primär, sekundär oder tertiär sein, bevorzugt sind solche mit primären oder sekundären und besonders bevorzugt mit primären Hydroxygruppen.

Primäre Hydroxygruppen sind Hydroxygruppen, die an ein Kohlenstoffatom gebunden sind, das mit genau einem weiteren Kohlenstoffatom verbunden ist. Analog ist bei sekundären Hydroxygruppen das an diese gebundene Kohlenstoffatom entsprechend mit zwei und bei tertiären Hydroxygruppen mit drei Kohlenstoffatomen verbunden.

Erfindungsgemäße Alkohole (A) sind primäre Alkohole der Formel (1) 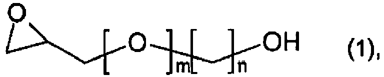 worin m eine ganze Zahl und 0 oder 1 ist und n ebenfalls eine ganze Zahl von 0 bis 10, bevorzugt von 1 bis 8, besonders bevorzugt von 1 bis 6 und insbesondere bevorzugt von 1 bis 4 ist, mit der Maßgabe, dass im Falle von m = 1 n ≠ 0 ist.

Beispiele für primäre Alkohole der Formel (1) sind 2,3-Epoxy-1-propanol (Glycidol), 3,4-Epoxy-1-butanol, 4,5-Epoxy-1-pentanol, 5,6-Epoxy-1-hexanol, Hydroxyethylgly-cidylether, Hydroxypropylglycidylether, Hydroxybutylglycidylether und Hydroxypentylglycidylether. Im Falle von n ≥ 5 können die Alkylreste auch verzweigt sein unter der Voraussetzung, dass es sich bei der Hydroxygruppe um eine primäre Hydroxygruppe handelt.

Bevorzugte Alkohole (A) sind 2,3-Epoxy-1-propanol (Glycidol), Hydroxyethylglycidylether und Hydroxybutylglycidylether.

Soweit die genannten Alkohole (A) optisch aktiv sind, werden sie bevorzugt racemisch oder als Diastereomerengemische eingesetzt, es ist jedoch auch möglich, sie als reine Enantiomere bzw. Diastereomere oder als Enantiomerengemische einzusetzen.

Im Reaktionsschritt erfolgt die Veresterung mit (Meth)acrylsäure (S) oder bevorzugt die Umesterung des Alkohols (A) mit mindestens einem (Meth)acrylsäureester (D) in Anwesenheit mindestens eines, bevorzugt eines die Umesterung katalysierenden Enzyms (E).

Im Falle der Umesterung ist es entscheidend, dass die alkoholische Abgangsgruppe, die bei der Umesterung vom (Meth)acrylsäureester (D) abgespalten wird, unter den Reaktionsbedingungen stabil ist und beispielsweise nicht isomerisiert. Das Reaktions-gleichgewicht kann daher auch noch auf die Seite der Edukte verschoben werden. Derartige (Meth)acrylsäureester (D) sind wie im Folgenden beschrieben beispielsweise Ester von gesättigten Alkoholen.

Zur Veresterung können (Meth)acrylsäure (S) oder zur Umesterung (Meth)acrylsäureester (D) eines gesättigten Alkohols eingesetzt werden, erfindungsgemäß sind gesättigte C₁ - C₁₀-Alkylester oder C₃ - C₁₂-Cycloalkylester der (Meth)acrylsäure, besonders bevorzugt gesättigte C₁ - C₄-Alkylester der (Meth)acrylsäure.

Gesättigt bedeutet im Rahmen dieser Schrift Verbindungen ohne C-C-Mehrfachbindungen (außer selbstverständlich die C=C-Doppelbindung in den (Meth)acryleinheiten).

Beispiele für (Meth)acrylsäureester (D) sind (Meth)acrylsäuremethyl-, -ethyl-, -n-butyl-, -iso-butyl-, n-octyl- und -2-ethylhexylester, 1,2-Ethylenglycoldi- und - mono(meth)acrylat, 1,4-Butandioldi- und -mono(meth)acrylat, 1,6-Hexandioldi- und-mono(meth)acrylat, Trimethylolpropantri(meth)acrylat und Pentaerythrittet-ra(meth)acrylat.

Besonders bevorzugt sind (Meth)acrylsäuremethyl-, -ethyl-, -n-butyl- und -2-Ethylhexylester.

Erfindungsgemäß einsetzbare Enzyme (E) sind ausgewählt aus Lipasen (E.C. 3.1.1.3) in freier oder auf einem Träger chemisch oder physikalisch immobilisierter Form. Ganz besonders bevorzugt sind Novozyme^{®} 435 (Lipase aus Candida antarctica B) oder Lipase aus Alcaligenes sp., Aspergillus sp., Mucor sp., Penicilium sp., Geotricum sp., Rhizopus sp., Burkholderia sp., Candida sp., Pseudomonas sp., Thermomyces sp. oder Schweinepankreas, insbesondere bevorzugt sind Lipase aus Candida antarctica B oder aus Burkholderia sp..

Der Enzymgehalt im Reaktionsmedium liegt in der Regel im Bereich von etwa 0,1 bis 10 Gew.-%, bezogen auf den eingesetzten Alkohol (A).

Die enzymatische Ver- bzw. Umesterung von (Meth)acrylsäure (S) bzw. von (Meth)acrylsäureestern (D) erfolgt im Allgemeinen bei 0 bis 100 °C, bevorzugt 20 bis 80 °C, besonders bevorzugt 20 bis 70 °C, ganz besonders bevorzugt 20 bis 60 °C.

Die Reaktionszeit hängt unter anderem von der Temperatur, der verwendeten Menge und der Aktivität des Enzymkatalysators und vom geforderten Umsatz ab sowie vom Alkohol. Bevorzugt wird die Reaktionszeit so angepasst, dass der Umsatz der umzusetzenden im Alkohol (A) enthaltenden, d.h. der niedrigersubstituierten Hydroxyfunktionen mindestens 70 %, bevorzugt mindestens 80, besonders bevorzugt mindestens 90, ganz besonders bevorzugt mindestens 95 %, insbesondere mindestens 97 % und speziell mindestens 98 % beträgt. In der Regel sind dafür 1 bis 72 Stunden, bevorzugt 3 bis 36 und besonders bevorzugt 3 bis 24 Stunden ausreichend.

Das molare Verhältnis von (Meth)acrylsäureverbindung (B) (bezogen auf die (Meth)acryleinheiten) zu Alkohol (A) (bezogen auf Hydroxygruppen) kann in einem weiten Bereich, wie z.B. im Verhältnis 100:1 bis 1:1, bevorzugt 50:1 bis 1:1, besonders bevorzugt 20:1 bis 1:1 und ganz besonders bevorzugt 10:1 bis 1:1, eingestellt werden.

Die Reaktion kann in organischen Lösungsmitteln oder deren Gemischen oder ohne Zusatz von Lösungsmitteln ablaufen. Bevorzugt wird kein Lösungsmittel zugesetzt. Die Ansätze sind in der Regel weitgehend wasserfrei (d.h. unter 10, bevorzugt unter 5, besonders bevorzugt unter 1 und ganz besonders bevorzugt unter 0,5 Vol-% Wasserzusatz).

Geeignete organische Lösungsmittel sind solche für diese Zwecke bekannten, beispielsweise tertiäre Monoole, wie C₃-C₆-Alkohole, bevorzugt tert.-Butanol, tert.-Amylalkohol, Pyridin, Poly-C₁-C₄-alkylenglykoldi-C₁-C₄-alkylether, bevorzugt Polyethy-lenglycoldi-C₁-C₄-alkylether, wie z.B. 1,2-Dimethoxyethan, Diethylenglycoldimethylether, Polyethylenglycoldimethylether 500, Methyl-tert.-butylether, Ethyl-tert.-butylether, C₁-C₄-Alkylencarbonate, insbesondere Propylencarbonat, C₃-C₆-Alkylessigsäureester, insbesondere tert.-Butylessigsäureester, Tetrahydrofuran, Toluol, 1,3-Dioxolan, Aceton, iso-Butylmethylketon, Ethylmethylketon, 1,4-Dioxan, tert.-Butylmethylether, Cyclohexan, Methylcyclohexan, Toluol, Hexan, Dimethoxymethan, 1,1-Dimethoxyethan, Acetonitril, sowie deren ein- oder mehrphasige Mischungen. Es kann vorteilhaft sein, freiwerdendes Wasser oder Alkohol durch ein möglichst nahe am Temperaturoptimum des verwendeten Enzyms (A) siedendes binäres oder ternäres Heteroazeotrop abzutrennen. Der so entfernte Alkohol kann dann durch Phasenscheidung oder Membrandampftrennung entfernt werden.

Wahlweise können zu den organischen Lösungsmitteln wässrige Lösungsmittel zugesetzt werden, so dass - je nach organischem Lösungsmittel - ein- oder mehrphasige Reaktionslösungen entstehen. Beispiele für wässrige Lösungsmittel sind Wasser sowie wässrige, verdünnte (z. B. 10 bis 100 mM) Puffer, beispielsweise mit einem pH-Wert im Bereich von etwa 6 bis 8, wie z.B. Kaliumphosphat- oder TRIS-HCl-Puffer.

Der Wasseranteil im Reaktionsansatz liegt in der Regel bei 0-10 Vol-%. Bevorzugt werden die Reaktanden ohne Vorbehandlung (Trocknung, Wasserdotierung) eingesetzt.

Die Substrate liegen entweder gelöst, als Feststoffe suspendiert oder in Emulsion im Reaktionsmedium vor. Vorzugsweise liegt die anfängliche Konzentration der Reaktanden im Bereich von etwa 0,1 bis 20 Mol/l, insbesondere bei 0,15 bis 10 Mol/l oder 0,2 bis 5 mol/l liegt.

Die Reaktion kann kontinuierlich, beispielsweise in einem Rohrreaktor oder in einer Rührreaktorkaskade, oder diskontinuierlich erfolgen.

Die Umsetzung kann in allen für eine solche Umsetzung geeigneten Reaktoren durchgeführt werden. Solche Reaktoren sind dem Fachmann bekannt. Bevorzugt erfolgt die Umsetzung in einem Rührkesselreaktor oder einem Festbettreaktor.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt und zum Start der Reaktion, sowie gegebenenfalls ein- oder mehrmals im Verlauf der Reaktion, mit dem Enzympräparat versetzt. Die Temperatur wird während der Reaktion auf den gewünschten Wert eingestellt und kann, falls gewünscht, während des Reaktionsverlaufs erhöht oder verringert werden.

Wird die Reaktion im Festbettreaktor durchgeführt, so ist der Festbettreaktor bevorzugt mit immobilisierten Enzymen bestückt, wobei die Reaktionsmischung durch eine mit dem Enzym gefüllte Säule gepumpt wird. Es ist auch möglich, die Umsetzung im Wirbelbett durchzuführen, wobei das Enzym auf einem Träger immobilisiert eingesetzt wird. Die Reaktionsmischung kann kontinuierlich durch die Säule gepumpt werden, wobei mit der Fließgeschwindigkeit die Verweilzeit und damit der gewünschte Umsatz steuerbar ist. Es ist auch möglich, die Reaktionsmischung im Kreislauf durch eine Säule zu pumpen, wobei auch unter Vakuum der freigesetzte Alkohol gleichzeitig abdestilliert werden kann.

Die Entfernung von Wasser im Falle einer Veresterung oder Alkoholen, die bei einer Umesterung aus den Alkyl(meth)acrylaten freigesetzt werden, erfolgt kontinuierlich oder schrittweise in an sich bekannter Weise, z. B. durch Destillation, Vakuum, azeotrope Entfernung, Absorption, Pervaporation und Diffusion über Membranen.

Hierzu eignen sich vorzugsweise Molekularsiebe oder Zeolithe (Porengröße z. B. im Bereich von etwa 3-10 Angström), eine Abtrennung durch Destillation oder mit Hilfe geeigneter semipermeabler Membranen.

Es ist aber auch möglich, das abgetrennte Gemisch aus Alkyl(meth)acrylat und dem diesem zugrundeliegenden Alkohol, das häufig ein Azeotrop bildet, direkt in eine Anlage zur Herstellung des Alkyl(meth)acrylats zuzuführen, um es dort in einer Veresterung mit (Meth)acrylsäure wiederzuverwerten.

Nach Beendigung der Reaktion kann man das aus der Ver- oder Umesterung erhaltene Reaktionsgemisch ohne weitere Aufreinigung weiterverwenden oder es erforderlichenfalls in einem weiteren Schritt aufreinigen.

In der Regel wird in einem Reinigungsschritt lediglich das eingesetzte Enzym vom Reaktionsgemisch abgetrennt und das Reaktionsprodukt vom gegebenenfalls verwendeten organischen Lösungsmittel abgetrennt.

Eine Abtrennung vom Enzym erfolgt in der Regel durch Filtration, Absorption, Zentrifugation oder Dekantieren. Das abgetrennte Enzym kann anschließend für weitere Re-aktionen eingesetzt werden.

Die Abtrennung vom organischen Lösungsmittel erfolgt in der Regel durch Destillation, Rektifikation oder bei festen Reaktionsprodukten durch Filtration.

Zur weiteren Aufreinigung des Reaktionsproduktes kann auch eine Chromatographie durchgeführt werden.

Bevorzugt werden im Reinigungsschritt jedoch lediglich das eingesetzte Enzym und das gegebenenfalls eingesetzte Lösungsmittel oder der Überschuss (Meth)acrylsäure bzw. (Meth)acrylat abgetrennt.

Die Reaktionsbedingungen bei der enzymatischen Ver- oder Umesterung sind mild. Aufgrund der niedrigen Temperaturen und sonstigen milden Bedingungen wird die Bil-dung von Nebenprodukten während der Reaktion vermieden, die andernfalls zum Beispiel von chemischen Katalysatoren stammen können oder durch unerwünschte radikalische Polymerisation des eingesetzten (Meth)acrylats, die sonst nur durch Zugabe von Stabilisatoren verhindert werden kann.

Bei der erfindungsgemäßen Reaktionsführung kann der (Meth)acrylverbindung (B) über den ohnehin enthaltenen Lagerstabilisator hinaus zusätzliche Stabilisatoren zugegeben werden, beispielsweise Hydrochinonmonomethylether, Phenothiazin, Phenole, wie z.B: 2-tert.-Butyl-4-methylphenol, 6-tert.-Butyl-2,4-dimethylphenol oder N-Oxyle, wie 4-Hydroxy-2,2,6,6-tetramethylpiperidin-N-oxyl, 4-Oxo-2,2,6,6-tetramethylpiperidin-N-oxyl, beispielsweise in Mengen von 50 bis 2000 ppm. Vorteilhaft wird die Ver- oder Umesterung in Gegenwart eines sauerstoffhaltigen Gases, bevorzugt Luft oder Luft-Stickstoff-Gemische, durchgeführt.

Des weiteren kann der Enzymkatalysator unproblematisch vom Endprodukt entfernt werden.

Das Reaktionsgemisch kann gegebenenfalls falls gewünscht aufgereinigt werden, beispielsweise durch Filtration, Destillation, Rektifikation, Chromatographie, Behandlung mit Ionentauschern, Adsorbentien, neutraler, saurer und/oder alkalischer Wäsche, Strippen oder Kristallisation.

Ein weiterer Gegenstand der vorliegenden Erfindung sind die aus den Alkoholen (A) durch enzymatische Ver- oder Umesterung erhaltenen (Meth)acrylsäureester (F). Diese weisen durch die erfindungsgemäßen Reaktionsbedingungen eine Farbzahl unter 100 APHA gemäß DIN ISO 6271, bevorzugt unter 80. Ferner enthalten sie in der Regel weniger als 1,0 % Nebenprodukte aus Umlagerungsreaktionen der Mehrfachbindung aus säure- oder basenkatalysierten Nebenreaktionen.

Der Vorteil der so nach dem erfindungsgemäßen Verfahren erhaltenen (Meth)acrylsäureester (F) ist der, dass sie wegen ihrer geringen Farbzahl in Lackanwendungen und dort insbesondere in Klarlacken vorteilhaft eingesetzt werden können, da sie durch ihre geringe Eigenfärbung eine verringerte Färbung der Beschichtungen gegenüber nach konventionellen Verfahren hergestellten Acrylaten bewirken.

Ferner können Beschichtungen mit den erfindungsgemäß hergestellten (Meth)acrylsäureestern (F) sehr hohe Kratzfestigkeiten, Härten, Chemikalienbeständigkeiten, Elastizität und Haftung, sowohl auf hydrophilen als auch auf hydrophoben Substraten aufweisen.

Die erfindungsgemäß erhältlichen (Meth)acrylsäureester (F) können vorteilhaft als Monomere oder Comonomere in Poly(meth)acrylaten oder als Reaktivverdünner in thermisch-, strahlungs- und/oder Dual-Cure-härtbaren Poly(meth)acrylaten eingesetzt werden. Derartige Poly(meth)acrylate sind beispielsweise als Bindemittel in thermisch-, strahlungs- oder Dual-Cure-härtbaren Beschichtungsmitteln sowie in Klebstoffen, z. B. Acrylatklebstoffen geeignet sowie in Dichtungsmassen.

Ein weiterer Gegenstand der vorliegenden Anmeldung ist daher die Verwendung der nach dem erfindungsgemäßen Verfahren hergestellten (Meth)acrylsäureester (F) als Reaktivverdünner oder Bindemittel in Strahlen oder Dual-Cure-härtbaren Beschichtungsmassen, bevorzugt in Deckbeschichtungen, besonders bevorzugt in transparenten Klarlacken. Selbstverständlich können die erfindungsgemäß hergestellten (Meth)acrylsäureester (F) auch als Monomere in Polymerisationen, gegebenenfalls zusammen mit anderen polymerisierbaren Monomeren, wie z. B. (Meth)acrylsäure, (Meth)acrylsäureester, Styrol, Butadien, Acrylnitril, Vinylacetat, N-Vinylpyrrolidon, 4-Hydroxybutylvinylether oder N-Vinylformamid, verwendet werden.

Unter "Dual-Cure" ist zu verstehen, dass die Beschichtungsmassen thermisch und mit aktinischer Strahlung härtbar sind. Im Rahmen der vorliegenden Erfindung ist unter aktinischer Strahlung elektromagnetische Strahlung wie sichtbares Licht, UV-Strahlung oder Röntgenstrahlung, insbesondere UV-Strahlung, und Korpuscularstrahlung wie Elektronenstrahlung zu verstehen.

Strahlenhärtbare Bindemittel sind solche, die mittels aktinischer Strahlung wie vorstehend definiert, insbesondere mittels UV-Strahlung härtbar sind.

Ein weiterer Gegenstand der vorliegenden Anmeldung sind Lackformulierungen, ent-haltend die nach dem erfindungsgemäßen Verfahren erhältlichen (Meth)acrylsäureester (F). Dabei können die (Meth)acrylsäureester (F) sowohl in Basislacken als auch in Decklacken eingesetzt werden. Aufgrund ihrer besonderen Eigenschaften, insbesondere ihrer geringen Farbzahl, ist ihr Einsatz in Deckbeschichtungen und einer strahlengehärteten Klarlackbeschichtungen bevorzugt.

Neben den nach dem erfindungsgemäßen Verfahren erhältlichen (Meth)acrylsäureester (F) kann eine erfindungsgemäße strahlungshärtbare Masse noch folgende Komponenten enthalten:
(G) mindestens eine polymerisierbare Verbindung mit mehreren copolymerisierbaren, ethylenisch ungesättigten Gruppen,
(H) gegebenenfalls Reaktivverdünner,
(I) gegebenenfalls Photoinitiator sowie
(J) gegebenenfalls weitere lacktypische Additive.

Als Verbindungen (G) kommen strahlungshärtbare, radikalisch polymerisierbare Verbindungen mit mehreren, d. h. mindestens zwei, copolymerisierbaren, ethylenisch ungesättigten Gruppen in Betracht.

Als Reaktivverdünner (Verbindungen (H)) kommen strahlungshärtbare, radikalisch oder kationisch polymerisierbare Verbindungen mit nur einer ethylenisch ungesättigten, copolymerisierbaren Gruppe in Betracht.

Als Photoinitiatoren (I) können dem Fachmann bekannte Photoinitiatoren verwendet werden, z. B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

Als weitere lacktypische Additive (J) können beispielsweise Antioxidantien, Oxidationsinhibitoren, Stabilisatoren, Aktivatoren (Beschleuniger), Füllmittel, Pigmente, Farbstoffe, Entgasungsmittel, Glanzmittel, antistatische Agentien, Flammschutzmittel, Verdicker, thixotrope Agentien, Verlaufshilfsmittel, Bindemittel, Antischaummittel, Duftstoffe, oberflächenaktive Agentien, Viskositätsmodifikatoren, Weichmacher, Plastifizierer, klebrigmachende Harze (Tackifier), Chelatbildner oder Verträglichkeitsmittel (compatibilizer) verwendet werden.

Beispiele für die genannten Verbindungsklassen (G), (H), (I) und (J) sind in WO 2006/005491 und in der unveröffentlichten deutschen Anmeldung mit dem Aktenzeichen DE 10 2005 037 430.1 offenbart. Auf beide Schriften wird an dieser Stelle ausdrücklich Bezug genommen.

Typische Zusammensetzungen für strahlungshärtbare Massen sind beispielsweise
(F) 20 - 100 Gew.-%, bevorzugt 40 - 90, besonders bevorzugt 50 - 90 und insbesondere 60 - 80 Gew.-%
(G) 0 - 60 Gew.-%, bevorzugt 5 - 50, besonders bevorzugt 10 - 40 und insbesondere 10 - 30 Gew.-%,
(H) 0 - 50 Gew.-%, bevorzugt 5 - 40, besonders bevorzugt 6 - 30 und insbesondere 10 - 30 Gew.-%,
(I) 0 - 20 Gew.-%, bevorzugt 0,5 - 15, besonders bevorzugt 1 - 10 und insbesondere 2 - 5 Gew.-% sowie
(J) 0 - 50 Gew.-%, bevorzugt 2 - 40, besonders bevorzugt 3 - 30 und insbesondere 5 - 20 Gew.-%,
mit der Maßgabe, dass (F), (G), (H), (I) und (J) zusammen 100 Gew.-% ergeben.

Die Beschichtung der Substrate erfolgt nach üblichen, dem Fachmann bekannten Verfahren, wobei man wenigstens eine Beschichtungsmasse auf das zu beschichtende Substrat in der gewünschten Stärke aufbringt und die gegebenenfalls enthaltenen flüchtigen Bestandteile der Beschichtungsmasse, gegebenenfalls unter Erhitzen, entfernt. Dieser Vorgang kann falls gewünscht ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Die Beschichtungsstärke liegt in der Regel in einem Bereich von etwa 3 bis 1000 g/m² und vorzugsweise 10 bis 200 g/m².

Weiterhin wird ein Verfahren zum Beschichten von Substraten offenbart, bei dem man die Beschichtungsmasse auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur.

Die Trocknung kann auch zusätzlich oder anstelle der thermischen Trocknung durch NIR-Strahlung erfolgen, wobei als NIR-Strahlung hier elektromagnetische Strahlung im Wellenlängenbereich von 760 nm bis 2,5 µm, bevorzugt von 900 bis 1500 nm bezeichnet ist.

Gegebenenfalls kann, wenn mehrere Schichten des Beschichtungsmittels übereinander aufgetragen werden, nach jedem Beschichtungsvorgang eine thermische und/oder NIR-Trocknung und Strahlungshärtung erfolgen.

Als Strahlungsquellen für die Strahlungshärtung geeignet sind z. B. Quecksilber-Niederdruckstrahler, -Mitteldruckstrahler und Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ = 200 bis 700 nm strahlt, besonders bevorzugt von λ = 200 bis 500 nm und ganz besonders bevorzugt λ = 250 bis 400 nm, oder durch Bestrahlung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z. B. zwei bis vier. Diese können auch in jeweils unterschiedlichen Wellenlängebereichen strahlen.

Die Bestrahlung kann gegebenenfalls auch unter Ausschluss von Sauerstoff, z. B. unter Inertgas-Atmosphäre, durchgeführt werden. Als Inertgase eignen sich vorzugsweise Stickstoff, Edelgase, Kohlendioxid, oder Verbrennungsgase. Des weiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE 199 57 900 A1 beschrieben ist.

Weiterhin sind auch Substrat, beschichtet mit einer erfindungsgemäßen Mehrschichtlackierung Gegenstand der vorliegenden Erfindung.

Die Dicke einer solche wie beschrieben zu härtenden Schicht kann von 0,1 µm bis mehrere mm betragen, bevorzugt von 1 bis 2000 µm, besonders bevorzugt 5 bis 1000 µm, ganz besonders bevorzugt von 10 bis 500 µm und insbesondere von 10 bis 250 µm.

Die erfindungsgemäß hergestellten (Meth)acrylsäureester (F) können aufgrund ihrer geringeren Färbung vorteilhaft auch in einer thermisch induzierten (radikalischen) (Co)polymerisation eingesetzt werden.

Als Monomere, mit denen die erfindungsgemäß hergestellten (Meth)acrylsäureester (F) beispielsweise copolymerisiert werden können seien genannt z.B. C₁-C₂₀-Alkyl(meth)acrylate, Vinylaromaten mit bis zu 20 C-Atomen, Vinylester von bis zu 20 C-Atomen enthaltenden Carbonsäuren, ethylenisch ungesättigte Nitrile, Vinylether von 1 bis 10 C-Atome enthaltenden Alkoholen und aliphatischen Kohlenwasserstoffen mit 2 bis 8 C-Atomen und 1 oder 2 Doppelbindungen.

Als (Meth)acrylsäurealkylester bevorzugt sind solche mit einem C₁-C₁₀-Alkylrest, wie Methylmethacrylat, Methylacrylat, n-Butylacrylat, Ethylacrylat und verzweigte Alkylderivate wie 2-Ethylhexylacrylat.

Insbesondere sind auch Mischungen der (Meth)acrylsäurealkylester geeignet.

Vinylester von Carbonsäuren mit 1 bis 20 C-Atomen sind z. B. Vinyllaurat, Vinylstearat, Vinylpropionat und Vinylacetat.

Als vinylaromatische Verbindungen kommen z. B. Vinyltoluol, α-Butylstyrol, 4-n-Butylstyrol, 4-n-Decylstyrol und vorzugsweise Styrol in Betracht.

Beispiele für Nitrile sind Acrylnitril und Methacrylnitril.

Geeignete Vinylether sind z. B. Vinylmethylether, Vinylisobutylether, Vinylhexylether und Vinyloctylether.

Als nicht aromatische Kohlenwasserstoffe mit 2 bis 8 C-Atomen und einer oder zwei olefinischen Doppelbindungen seien Butadien, Isopren, sowie Ethylen, Propylen und Isobutylen genannt.

Eine häufige, aber nicht die einzige Methode zur Herstellung solcher (Co)Polymerisate ist die radikalische oder ionische (Co)Polymerisation in einem Lösungs- oder Verdünnungsmittel.

Die radikalische (Co)Polymerisation solcher Monomere erfolgt beispielsweise in wässriger Lösung in Gegenwart von Polymerisationsinitiatoren, die unter Polymerisations-bedingungen in Radikale zerfallen, beispielsweise Peroxodisulfate, H₂O₂-Redoxsysteme oder Hydroperoxide, wie z. B. tert.-Butylhydroperoxid oder Cumolhydroperoxid. Die (Co)Polymerisation kann in einem weiten Temperaturbereich, gegebenenfalls unter vermindertem oder auch unter erhöhtem Druck in der Regel bei Temperaturen bis zu 100 °C vorgenommen werden. Der pH-Wert des Reaktionsgemisches wird gewöhnlich in dem Bereich von 4 bis 10 eingestellt.

Die (Co)Polymerisation kann aber auch in anderer, dem Fachmann an sich bekannter Weise kontinuierlich oder diskontinuierlich durchgeführt werden, z. B. als Lösungs-, Fällungs-, Wasser-in-Öl-Emulsions-, inverse Emulsions-, Suspensions- oder umgekehrte Suspensionspolymerisation.

Dabei wird das Monomer/die Monomere unter Verwendung radikalischer Polymerisati-onsinitiatoren, z. B. in Radikale zerfallende Azoverbindungen, wie 2,2'-Azo-bis(isobutyronitril), 2,2'-Azobis-(2-amidinopropan)-hydrochlorid oder 4,4'-Azo-bis-(4'-cyanpentansäure) oder Dialkylperoxide, wie Di-tert.-amylperoxid, Arylalkylperoxide, wie tert.-Butylcumylperoxid, Alkylacylperoxide, wie tert.-Butylperoxy-2-ethylhexanoat, Peroxidicarbonate, wie Di-(4-tert.-Butylcyclohexyl)peroxydicarbonat oder Hydroperoxide (co)polymerisiert.

Die genannten Verbindungen werden meist in Form wässriger Lösungen oder wässriger Emulsionen eingesetzt, wobei die untere Konzentration durch die in der (Co)Polymerisation vertretbare Wassermenge und die obere Konzentration durch die Löslichkeit der betreffenden Verbindung in Wasser bestimmt ist.

Als Lösungs- oder Verdünnungsmittel können dienen z. B. Wasser, Alkohole, wie Methanol, Ethanol, n- oder iso-Propanol, n- oder iso-Butanol, oder Ketone, wie Aceton, Ethylmethylketon, Diethylketon oder iso-Butylmethylketon. Besonders bevorzugt sind unpolare Lösungsmittel wie beispielsweise Xylol und dessen Isomerengemische, Shellsol^{®} A und Solventnaphtha.

In einer bevorzugten Ausführungsform werden die Monomere vorgemischt und Initiator mit gegebenenfalls weiteren Zusätzen gelöst in Lösungsmittel zugegeben. Eine besonders bevorzugte Ausführungsform ist beschrieben in WO 2001/23484 und dort besonders auf Seite 10, Z. 3 bis Z. 24.

Gegebenenfalls kann die (Co)Polymerisation in Gegenwart von Polymerisationsreglern, wie beispielsweise Hydroxylammoniumsalze, chlorierte Kohlenwasserstoffe und Thioverbindungen, wie z.B. tert.-Butylmercaptan, Thioglycolsäureethylacrylester, Mercaptoethynol, Mercaptopropyltrimethoxysilan, Dodecylmercaptan, tert.-Dodecylmercaptan oder Alkalimetallhypophosphite, durchgeführt werden. Bei der (Co)Polymerisation können diese Regler, z. B. in Mengen von 0 bis 0,8 Gew.-Teile, bezogen auf 100 Gew.-Teile der zu (co)polymerisierenden Monomeren, eingesetzt werden, durch die die Molmasse des entstehenden (Co)Polymers verringert wird.

Bei der Emulsionspolymerisation können Dispergiermittel, ionische und/oder nichtionische Emulgatoren und/oder Schutzkolloide bzw. Stabilisatoren als grenzflächenaktive Verbindungen verwendet werden. Als solche kommen sowohl die zur Durchführung von Emulsionspolymerisationen üblicherweise eingesetzten Schutzkolloide als auch Emulgatoren in Betracht.

Geeignete Schutzkolloide sind beispielsweise Polyvinylalkohole, Cellulosederivate oder Vinylpyrrolidon enthaltende Copolymerisate. Eine ausführliche Beschreibung weiterer geeigneter Schutzkolloide findet sich in Houben-Weyl, Methoden der organischen Chemie, Band XIV/1, makromolekulare Stoffe, Georg-Thieme-Verlag, Stuttgart, 1969, S. 411 bis 420. Selbstverständlich können auch Gemische aus Emulgatoren und/oder Schutzkolloiden verwendet werden. Vorzugsweise werden als Dispergiermittel ausschließlich Emulgatoren eingesetzt, deren relative Molekulargewichte im Unterschied zu den Schutzkolloiden üblicherweise unter 1.000 liegen. Sie können sowohl anionischer, kationischer oder nichtionischer Natur sein. Selbstverständlich müssen im Falle der Verwendung von Gemischen grenzflächenaktiver Substanzen die Einzelkomponenten miteinander verträglich sein, was im Zweifelsfall an Hand weniger Vorversuche überprüft werden kann. Im allgemeinen sind anionische Emulgatoren untereinander und mit nichtionischen Emulgatoren verträglich.

Desgleichen gilt auch für kationische Emulgatoren, während anionische und kationische Emulgatoren meistens miteinander unverträglich sind. Gebräuchliche Emulgatoren sind z. B. ethoxylierte Mono-, Di- und Tri-Alkylphenole (EO-Grad: 3 bis 100, Alkylrest: C₄ bis C₁₂), ethoxylierte Fettalkohole (EO-Grad: 3 bis 100, Alkylrest: C₈ bis C₁₈), sowie Alkali- und Ammoniumsalze von Alkylsulfaten (Alkylrest: C₈ bis C₁₆) von Schwefelsäurehalbestern ethoxylierter Alkylphenole (EO-Grad: 3 bis 100, Alkylrest: C₄ bis C₁₂), von Alkylsulfonsäuren (Alkylrest: C₁₂ bis C₁₈) und von Alkylacrylsulfonsäuren (Alkylrest: C₉ bis C₁₈). Weitere geeignete Emulgatoren wie Sulfobernsteinsäureester finden sich in Houben-Weyl, Methoden der organischen Chemie, Band XIV/1, Makromolekulare Stoffe, Georg-Thieme Verlag, Stuttgart, 1961, Seiten 192 bis 208.

In der Regel beträgt die Menge an eingesetzten Dispergiermittel 0,5 bis 6, vorzugsweise 1 bis 3 Gew.-% bezogen auf die radikalisch zu polymerisierenden Monomeren.

Beispiele für (meth)acrylathaltige Dispersionen sind n-Butylacrylat/Acrylnitril - Dispersionen, die als Klebstoffe Anwendung finden, sowie n-Butylacrylat/Butadien/Styrol - Dispersionen.

Die Polymerdispersionen, in denen erfindungsgemäß hergestellte (Meth)acrylsäureester (F) verwendet werden, können zusätzlich chemisch und/oder physikalisch desodoriert werden.

Eine chemische Desodorierung kann beispielsweise wie von P.H.H. Araüjo, C. Sayer, J. G. R. Poco, R. Giudici, in Polymer Engineering and Science, 2002 (42), 1442-1468 beschrieben oder in EP 1 375 530 B1 offenbart, durchgeführt werden.

Die mit den erfindungsgemäß hergestellten (Meth)acrylsäureestern (F) erhältlichen Copolymerisate weisen in der Regel eine geringere Farbzahl auf, was im Lackbereich vorteilhaft ist. Die beschriebenen Copolymerisate lassen sich dann in an sich bekannter Weise beispielsweise mit Aminoplasten, wie z. B. Melamin, zu vernetzten Lackharzen umsetzen, wie es beispielsweise beschrieben ist in der EP 0 738 740 oder EP 0 675 141.

Besonders bevorzugt eignen sich die erfindungsgemäßen Beschichtungsmassen als oder in Außenbeschichtungen, also solche Anwendungen, die dem Tageslicht ausgesetzt sind, bevorzugt von Gebäuden oder Gebäudeteilen, Innenbeschichtungen, Straßenmarkierungen, Beschichtungen auf Fahrzeugen und Flugzeugen. Insbesondere werden die Beschichtungen als Holz-, Papier- oder Kunststoffbeschichtungen, beispielsweise für Parkett oder Möbel eingesetzt.

Ein weiterer Gegenstand ist die Verwendung der erfindungsgemäß erhaltenen Produkte als Vorprodukt für Glanzzusätze in der Galvanotechnik. Durch ihre gegenüber konventionell erhältlichen Produkten verringerte Farbzahl macht sie für diese Anwendung außerordentlich geeignet.

Mit Hilfe des erfindungsgemäßen Verfahrens ist die Herstellung (Meth)acrylsäureester (F) in hoher chemischer und Raum-Zeit-Ausbeute und unter milden Bedingungen mit guten Farbzahlen möglich. Trotz Verzicht auf aktivierte (Meth)acrylsäureverbindungen werden gezielt die gewünschten Produkte mit hoher Selektivität erhalten, die weitgehend frei von Nebenprodukten sind.

Die folgenden Beispiele sollen die Eigenschaften der Erfindung erläutern, ohne sie aber einzuschränken.

### Beispiele

Als "Teile" seien in dieser Schrift, wenn nicht anders angegeben, "Gewichtsteile" verstanden.

### Beispiel 1

### Herstellung von Glycidolacrylat in Lösungsmittel

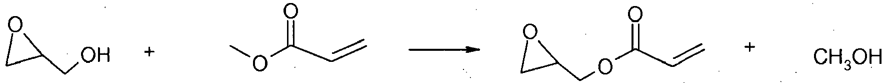

In einem Schraubdeckelglas wurden jeweils 5 mMol Glycidol (370 mg) mit 10 bzw. 50 mMol Methylacrylat, 50 mg Novozym^{®} 435 (geträgerte Lipase aus Candida antarctica B, Fa. Novozymes, Dänemark), 5,0 ml MTBE (tert.-Butylmethylether) und evtl. 1,0 g Molekularsieb 5 Å für 24 h bei 20 bzw. 40 °C geschüttelt. Danach wurde das Enzym abfiltriert und der Überschuss Methylacrylat am Rotationsverdampfer entfernt. Man erhielt ein farbloses Acrylat.

Zur Bestimmung des Umsatzes wurde eine Probe silyliert und mittels GC der Umsatz von Alkohol zu Acrylat bestimmt. Es wurden keine Nebenprodukte mit einem Gehalt von > 0,2 % gefunden.

**Umsätze [%] bei 20 °C.**

| Methacrylat [mMol] | Ohne MS | Mit MS |
|---|---|---|
| 10 | 47 | 70 |
| 50 | 74 | 92 |
| 50 | - | 64 |

| | | |
|---|---|---|
| * MS = Molekularsieb 5 Å ^{a} mit Lipase aus Burkholderia plantarii anstelle von Novozym^{®} 435 | | |

**Umsätze [%] bei 40 °C**

| Methacrylat [mMol] | Ohne MS | Mit MS |
|---|---|---|
| 10 | 49 | 79 |
| 50 | 76 | > 99 |

| | | |
|---|---|---|
| * MS = Molekularsieb 5 Å | | |

### Beispiel 2

### Herstellung von 4-Hydroxybutylacrylatglycidylether (4-HBAGE) mit unterschiedlichem Überschuss Methylacrylat

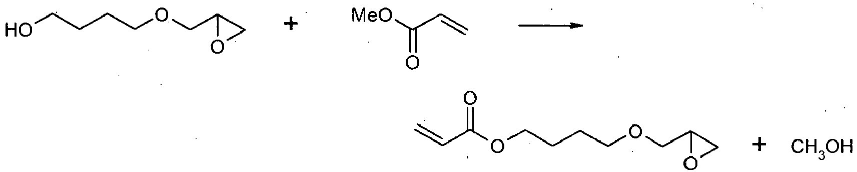

In einem Schraubdeckelglas wurden jeweils 5 mMol 4-Hydroxybutylglycidylether (731 mg) mit 20, 30, 40 bzw. 50 mMol Methylacrylat, 0 bzw. 25 mg Novozym 435 (geträgerte Lipase aus Candida antarctica B, Fa. Novozymes, Dänemark) und evtl. 1,0 g Molekularsieb 5 Å für 24 h bei 40 °C geschüttelt. Danach wurde das Enzym abfilt-riert und der Überschuss Methylacrylat am Rotationsverdampfer entfernt. Man erhielt ein farbloses Acrylat.

Zur Bestimmung des Umsatzes wurde eine Probe silyliert und mittels GC der Umsatz von Alkohol zu Acrylat bestimmt. Es wurde keine Nebenprodukte mit einem Gehalt von > 0,2 % gefunden.

| Methylacrylat [mMol] | Anmerkung | Umsatz [%] |
|---|---|---|
| 50 | ohne Enzym | 0 |
| 50 | Ohne Molekularsieb | 29 |
| 50 | - | 100 |
| 40 | - | 100 |
| 30 | - | 100 |
| 20 | - | 100 |

### Beispiel 3

### Herstellung von 4-Hydroxybutylacrylatglycidylether (4-HBAGE), Einfluss von Temperatur und Reaktionszeit

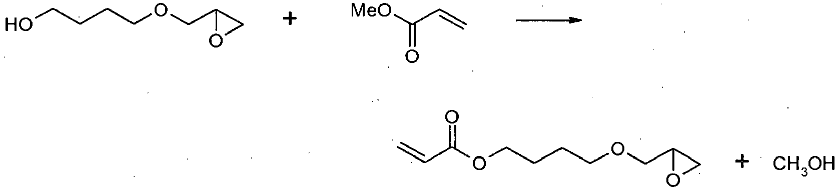

Es wurden jeweils 20 mMol 4-Hydroxybutylglycidylether (2,92 g) mit 80 mMol Methylacrylat (6,89 g), 100 mg Novozym^{®} 435 und 4,0 g Molekularsieb 5 Å für 2, 4, 6, 8 bzw. 24 h bei 20 bzw. 40 °C gerührt. Danach wurde das Enzym abfiltriert und der Überschuss Methylacrylat am Rotationsverdampfer entfernt. Man erhielt ein farbloses Acrylat mit 93 % (bei 20 °C) bzw. 94 % (bei 40 °C) Ausbeute.

Zur Bestimmung des Umsatzes wurde jeweils eine Probe silyliert und mittels GC der Umsatz von Alkohol zu Acrylat bestimmt. Es wurde keine Nebenprodukte mit einem Gehalt von > 0,2 % gefunden.

| Reaktionszeit [h] | Umsatz [%] bei 20 °C | Umsatz [%] Bei 40 °C |
|---|---|---|
| 2 | 16 | 45 |
| 4 | 33 | 78 |
| 6 | 49 | 97 |
| 8 | 62 | 100 |
| 24 | 100 | 100 |

## Patentansprüche

1. Verfahren zur Herstellung von (Meth)acrylsäureestern (F) von mindestens eine Epoxygruppe aufweisenden Alkoholen (A), **dadurch gekennzeichnet, dass** man mindestens einen mindestens eine Epoxygruppe aufweisenden Alkohol (A) in Gegenwart mindestens eines Enzyms (E) mit (Meth)acrylsäure (S) verestert oder mit mindestens einem (Meth)acrylsäureester (D) umestert, wobei im Falle der Umesterung die alkoholische Abgangsgruppe unter den Reaktionsbedingungen stabil ist, wobei es sich bei dem (Meth)acrylsäureester (D) um einen gesättigten C₁ - C₁₀-Alkylester handelt, es sich bei dem Epoxygruppen aufweisenden Alkohol (A) um einen der Formel (1) 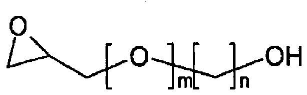 handelt,
worin m eine ganze Zahl und 0 oder 1 ist und n ebenfalls eine ganze Zahl von 0 bis 10, mit der Maßgabe, dass im Falle von m = 1 n ≠ 0 ist und das Enzym (E) ausgewählt ist aus der Gruppe der Lipasen (E.C. 3.1.1.3).

2. Verfahren nach Anspruch 1, **dadurch gekennzeichnet, dass** die Hydroxygruppe der Epoxygruppen aufweisenden Alkohole (A) primär ist.

3. Verfahren nach einem der vorstehenden Ansprüche, **dadurch gekennzeichnet, dass** der Epoxygruppen aufweisende Alkohol ausgewählt ist aus der Gruppe bestehend aus 2,3-Epoxy-1-propanol, Hydroxyethylglycidylether und Hydroxybutylglycidylether.

4. Verfahren nach einem der vorstehenden Ansprüche, **dadurch gekennzeichnet, dass** der (Meth)acrylsäureester (D) ausgewählt ist aus der Gruppe bestehend aus (Meth)acrylsäuremethylester, (Meth)acrylsäureethylester, (Meth)acrylsäuren-butylester und (Meth)acrylsäure-2-ethylhexylester.

## Claims

1. A process for preparing (meth)acrylic esters (F) of alcohols (A) having at least one epoxy group, which comprises esterifying at least one alcohol (A) having at least one epoxy group with (meth)acrylic acid (S) or transesterifying it with at least one (meth)acrylic ester (D) in the presence of at least one enzyme (E), the alcoholic leaving group being stable under the reaction conditions in the case of the transesterification, the (meth)acrylic ester (D) being a saturated C₁-C₁₀-alkyl ester, the alcohol (A) having epoxy groups being one of the formula (1) 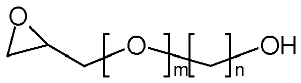 in which m is an integer and is 0 or 1 and n is likewise an integer of from 0 to 10, with the proviso that, in the case that m = 1, n ≠ 0, and the enzyme (E) being selected from the group of the lipases (E.C. 3.1.1.3).

2. The process according to claim 1, wherein the hydroxyl group of the alcohols (A) having epoxy groups is primary.

3. The process according to either of the preceding claims, wherein the alcohol having epoxy groups is selected from the group consisting of 2,3-epoxy-1-propanol, hydroxyethyl glycidyl ether and hydroxybutyl glycidyl ether.

4. The process according to any of the preceding claims, wherein the (meth)acrylic ester (D) is selected from the group consisting of methyl (meth)acrylate, ethyl (meth)acrylate, n-butyl (meth)acrylate and 2-ethylhexyl (meth)acrylate.

## Revendications

1. Procédé de fabrication d'esters de l'acide (méth)acrylique (F) d'alcools (A) comprenant au moins un groupe époxy, **caractérisé en ce qu'**au moins un alcool (A) comprenant au moins un groupe époxy est estérifié en présence d'au moins une enzyme (E) avec de l'acide (méth)acrylique (S) ou transestérifié avec au moins un ester de l'acide (méth)acrylique (D), le groupe de départ alcoolique étant stable dans les conditions de réaction dans le cas de la transestérification, l'ester de l'acide (méth) acrylique (D) étant un ester alkylique en C₁-C₁₀ saturé, l'alcool (A) comprenant des groupes époxy étant un de formule (1) 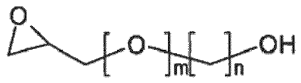 dans laquelle m est un nombre entier et représente 0 ou 1, et n est également un nombre entier de 0 à 10, à condition que lorsque m = 1, n soit ≠ 0, et l'enzyme (E) est choisie dans le groupe des lipases (E. C. 3.1.1.3).

2. Procédé selon la revendication 1, **caractérisé en ce que** le groupe hydroxy des alcools (A) comprenant des groupes époxy est primaire.

3. Procédé selon l'une quelconque des revendications précédentes, **caractérisé en ce que** l'alcool comprenant des groupes époxy est choisi dans le groupe constitué par le 2,3-époxy-1-propanol, l'éther glycidylique d'hydroxyéthyle et l'éther glycidylique d'hydroxybutyle.

4. Procédé selon l'une quelconque des revendications précédentes, **caractérisé en ce que** l'ester de l'acide (méth)acrylique (D) est choisi dans le groupe constitué par l'ester méthylique de l'acide (méth)acrylique, l'ester éthylique de l'acide (méth)acrylique, l'ester n-butylique de l'acide (méth)acrylique et l'ester 2-éthylhexylique de l'acide (méth)acrylique.
